Europäisches Patentamt

European Patent Office

Office européen des brevets

(11) Veröffentlichungsnummer: **0 482 727 A2**

## EUROPÄISCHE PATENTANMELDUNG

(21) Anmeldenummer: **91250290.3**

(22) Anmeldetag: **24.10.91**

(51) Int. Cl.5: **G01N 33/543**, G01N 33/66, G01N 33/58

(30) Priorität: **26.10.90 DE 4034565**

(43) Veröffentlichungstag der Anmeldung:
**29.04.92 Patentblatt 92/18**

(84) Benannte Vertragsstaaten:
**AT CH FR GB IT LI NL SE**

(71) Anmelder: **INSTITUT FUR HALBLEITERPHYSIK**
**Walter-Korsing-Strasse 2**
**O-1200 Frankfurt/Oder(DE)**

(72) Erfinder: **Ehwald, Karl-Ernst**
**Clara-Zetkin-Ring 45**
**O-1200 Frankfurt (Oder)(DE)**
Erfinder: **Knoll, Dieter, Dr.**
**Betonstrasse 2**
**O-1211 Falkenhagen(DE)**
Erfinder: **Ehwald, Rudolf, Prof.**
**Egon-Schulz-Strasse 6**
**O-1040 Berlin(DE)**
Erfinder: **Abel, Peter, Dr.**
**Dorfstrasse 2**
**O-2204 Züssow(DE)**

(54) **Stereospezifischer Sensor für die Bestimmung von Zuckern.**

(57) Der Sensor registriert die Konkurrenz eines hochmolekularen, geeignete Zuckerreste tragenden Liganden mit dem in seiner Konzentration zu erfassenden Zucker um die Zuckerbindungsorte eines Lektins mit Hilfe einer Strahlenmeßsonde. Das Lektin oder ein hochmolekularer Ligand mit lektinspezifischen Zuckerresten sind in einer Diffusionskammer in einer lokal begrenzten Zone in unlöslicher Form fixiert. Der ebenfalls in der Diffusionskammer befindliche komplementäre diffusible hochmolekulare Bindungspartner ist durch ein Radioisotop markiert und befindet sich in der Kammer im diffusiblen oder in der lokal begrenzten Zone im austauschbar gebundenen Zustand. An oder in der Kammer befindet sich eine Meßsonde für radioaktive Strahlung. Die Diffusionskammer kann mit Wasser oder einer wäßrigen Lösung gefüllt werden und mit der zu untersuchenden Lösung über eine semipermeable Dialysemembran austauschen. Die semipermeable Dialysemembran ist für Zucker durchlässig, für das eingeschlossene diffusible radioaktiv markierte Lektin oder den diffusiblen hochmolekularen radioaktiv markierten Lektin-Bindungspartner dagegen vollkommen undurchlässig. Die Dissoziation zwischen dem Lektin und dem Liganden ist abhängig von der Konzentration des Zuckers in der zu untersuchenden Lösung und führt zu einer räumlichen Verlagerung des radioaktiv markierten hochmolekularen Bindungspartners. Der hierdurch veränderte Quench wird mit der Strahlenmeßsonde registriert.

Rank Xerox (UK) Business Services
(-/2.17/2.0)

Die vorliegende Erfindung betrifft einen stereospezifischen Sensor für die Bestimmung von Zuckern. Insbesondere kann er auch für die in vivo Blutzuckermessung benutzt werden.

Stereospezifische elektronische Konzentrationsmeßsonden für die Bestimmung von Zuckern sind auf der Grundlage zuckerspezifischer Enzyme seit langem bekannt. Sie besitzen große Bedeutung für die Medizin und die Biotechnologie. Leider ist die Zahl der für die Zuckerbestimmung verfügbaren Redoxenzymsysteme begrenzt, so daß für zahlreiche Zucker überhaupt noch keine elektronischen Sensoren zur Verfügung stehen. Bei weitem die größte Bedeutung haben Elektroden für die Bestimmung der Glucose mit Hilfe der Glucose-Oxidase.

Obwohl die Glucosebestimmung mit der Enzymelektrode grundsätzlich für den Blutzucker-Konzentrationsbereich erfolgreich durchgeführt werden kann, ist die Langzeitanwendung der Enzymelektroden für die in vivo-Blutzuckerbestimmung nach wie vor problematisch. Verschiedene Erfindungen auf diesem Gebiet betreffen die Miniaturisierung sowie die Vermeidung von Störeinflüssen auf das Meßsignal und Veränderungen in der Empfindlichkeit. Ein grundsätzliches Problem für die Blutzuckermessung in vivo mit Hilfe von Enzymelektroden besteht in der Irreversibilität der in Frage kommenden Enzymreaktionen, wodurch es unumgänglich wird, das elektrische Meßsignal durch Wandlung eines chemischen Flusses zu erzeugen. Die hierbei auftretenden Diffusionsvorgänge sind unter in vivo-Bedingungen schwer zu standardisieren und konstant zu halten.

Die Aufgabe besteht in der Bereitstellung eines miniaturisierbaren und stereospezifischen elektrischen Sensors für Zucker, der im Unterschied zu den Enzymelektroden auf einem konzentrationsabhängigen Signal beruht, das ein thermodynamisches Gleichgewicht charakterisiert und dementsprechend ohne Zuckerverbrauch zustande kommt.

Die Aufgabe wird dadurch gelöst, daß der Sensor die Konkurrenz eines hochmolekularen, geeignete Zuckerreste tragenden Liganden mit dem in seiner Konzentration zu erfassenden Zucker um die Zuckerbindungsorte eines Lektins mit Hilfe einer Strahlenmeßsonde registriert. Das Lektin oder ein hochmolekularer Ligand mit lektinspezifischen Zuckerresten ist in einer Diffusionskammer in einer oder mehreran lokal begrenzten Zonen in unlöslicher Form fixiert. Der ebenfalls in der Diffusionskammer befindliche komplementäre diffusible hochmolekulare Bindungspartner ist durch ein Radioisotop markiert und befindet sich in der Kammer im diffusiblen oder in der(n) lokal begrenzten Zone(n) austauschbar gebundenen Zustand. An oder in der Kammer befindet sich eine Meßsonde für radioaktive Strahlung. Die Diffusionskammer kann mit Wasser oder einer wäßrigen Lösung gefüllt werden und mit der zu untersuchenden Lösung über eine semipermeable Dialysemembran austauschen. Die semipermeable Dialysemembran ist für Zucker durchlässig, für das eingeschlossene diffusible radioaktive Lektin oder den diffusiblen hochmolekularen Lektin-Bindungspartner dagegen vollkommen undurchlässig. Die Dissoziation zwischen dem Lektin und dem Liganden ist abhängig von der Konzentration des Zuckers in der zu untersuchenden Lösung und führt zu einer räumlichen Verlagerung des radioaktiv markierten hochmolekularen Bindungspartners. Der hierdurch veränderte Quench wird mit der Strahlenmeßsonde registriert.

Für die Miniaturisierung besonders geeignet ist eine Strahlenmeßsonde, welche, als Festkörpersensor ausgebildet, die strahlungsinduzierte Ionisierung entweder in halbleitenden oder isolierenden Materialien zur Erzeugung eines dar Strahlungsintensität proportionalen elektrischan Signales ausnutzt. Dieses an sich bekannte Funktionsprinzip, zum Beispiel technisch realisiert in Form eines in Sperrichtung vorgespannten pn-Überganges in einem Halbleitermaterial, in dessen Raumladungszone durch die Strahlung Ladungsträger erzeugt werden oder in Form einer Kondensatoranordnung, in deren Dielektrikum durch die Strahlung bei angelegter, ausreichend hoher Spannung ein Stromfluß zustande kommt, erfordert für die Funktion zur Gewährleistung einer ausreichenden Empfindlichkeit des Sensors eine hohe Versorgungsspannung (ca. 100 V). Diese Forderung erweist sich vor allem in Hinsicht auf eine in vivo-Anwendung des Sensors als sehr störend. Außerdem ist das unmittelbare Ausgangssignal eines solchen Sensors selbst bei genügend hoher Betriebsspannung wegen der in gleicher Hinsicht gering zu haltenden Radioaktivität so klein, daß es auf Grund kaum zu lösender Isolationsprobleme für eine Weiterverwendung, zum Beispiel zur Ansteuerung einer externen Anzeige oder als Triggersignal für ein Stellglied in einem Regelkreis zur Konstanthaltung des Blutzuckerspiegels, nicht nutzbar ist.

Die Aufgabe besteht somit in der Bereitstellung einer kompakten, möglichst monolithischen Sensorfunktionseinheit, in der sowohl die unmittelbare Wandlung der ein Maß für die zu bestimmende Zuckerkonzentration darstellenden Strahlungsintensität in ein elektrisches Signal als auch die Bereitstellung einer genügend hohen Betriebsspannung aus einer externen niedrigen Versorgungsspannung sowie die Selektion und Verstärkung des elektrischen Nutzsignals vor seiner Herausführung aus dem unmittelbaren Sensorbereich zur Weiterverarbeitung gewährleistet wird. Anzustreben ist dabei ferner die konstruktive Vereinigung der Festkörperstrahlungssonde mit der Diffusionskammer zu einer mechanisch stabilen Funktionseinheit. Die Aufgabe wird dadurch gelöst, daß mit den Mitteln der Silizium-CMOS- oder -BICMOS-Technologie ein monolithischer, extrem miniaturisierbarer Funktionsblock hergestellt wird, der die Funktionselemente-Wandlung der Strahlungsintensität in ein elektrisches Signal in einem in Sperrichtung vorgespannten pn-Übergang und/oder in einer Kondensatoranordnung mit $SiO_2$ als Dielektrikum interne Spannungserzeugung durch Kaskadenverstärkung einer niedrigen externen Versorgungsspannung sowie Umformung und Verstärkung das zuckerkonzentrationsproportionalen elektrischen Signals enthält.

Entscheidend für die Funktion der Anordnung besonders hinsichtlich ihrer Langzeitstabilität ist eine ausreichende Abkopplung der Schaltungsbestandteile, die nicht funktionsbedingt der Strahlung ausgesetzt werden müssen, vom Strahlungsfeld und die Beschichtung der Oberfläche des strahlungsempfindlichen Teils des integrierten Bausteins mit biokompatiblen bzw. mit einer mit der Kammerflüssigkeit nicht reagierenden Schicht. In diesem Falle kann der Sensor und die Diffusionskammer mit der Dialysemembran und gegebenenfalls mit dem Lektin- bzw. Ligandenträger zu einer mechanisch stabilen und dichten Funktionseinheit vereinigt werden.

Bei dem erfindungsgemäßen Sensor stammt die Primärenergie für das Zustandekommen des Signals aus dem radioaktiven Zerfall eines langlebigen Isotops. Die Halbwertzeit des Isotops $^{14}C$ ist ausreichend hoch, um eine für praktische. Zwecke ausreichende Langzeitkonstanz der eingeschlossenen Radioaktivität zu gewährleisten. An die semipermeable Membran wird bei diesem Sensor die Anforderung gestellt, daß sie für kleine Moleküle wie Zucker (Molmasse 180 D) hochpermeabel, für radioaktive Lektinmoleküle oder diffusible hochmolekulare radioaktive Lektinliganden vollkommen unpermeabel ist. Im Fall des für einen Glucose-Sensor geeigneten Concanavallin-A oder eines ebenfalls für den Glucose-Sensor geeigneten hochmolekularen Dextrans ist diese Voraussetzung ohne Schwierigkeit bei zahlreichen biokompatiblen und stabilen Membranmaterialien (z. B. auf der Basis von Polysulfonat oder Celluloseacetat) erfüllbar. Bei entsprechender Miniaturisierung der Meßkammer werden nur sehr geringe Mengen des Radioisotops benötigt (im nCurie- bis μCurie-Bereich). Das Radioisotop bleibt in der Diffusionskammer eingeschlossen. Die geringe Energie der β-Strahlen gewährleistet im Fall von $^{14}C$ einerseits eine gute Modulation der Zählausbeute durch Verlagerung des markierten Stoffes in der Kammer und andererseits eine sehr geringe Strahlenbelastung, besonders, wenn die Kammer ein Gehäuse aus Metall oder Edelmetall besitzt.

Mit der von der Zuckerkonzentration abhängigen Verteilung der Sondenmoleküle im gesamten Kammervolumen ergeben sich konzentrationsabhängige Quench-Bedingungen und eine entsprechend veränderte Zählausbeute am Strahlensensor. Die konzentrationsabhängige Veränderung der Zählausbeute kann man durch die konstruktive Gestaltung der Kammer, zum Beispiel durch den Einbau von elektronendichten Abschirm-Strukturen, verstärken.

Der wesentliche Vorteil des erfindungsgemäßen Sensors liegt darin, daß die hohe Stereospezifität der Lektin-Bindung an den Zucker ohne einen irreveribel verlaufenden chemischen Prozeß ausnutzbar ist, wobei im Unterschied zu den enzymatischen Verfahren ein reversibler Vorgang der kompetitiven Verdrängung der Lektinliganden vom Lektin der Signalmodulation zugrunde liegt. Ein weiterer Vorteil der Erfindung liegt in der großen Zahl verfügbarer Lektine, die es ermöglicht, praktisch für jeden natürlich vorkommenden Zucker einen stereospezifischen elektronischen Sensor herzustellen. Bei richtiger Wahl der Kammerdimension erfolgt die diffusionslimitierte Gleichgewichtseinstellung nach Veränderung der Zuckerkonzentration auch ohne zusätzliche Rührvorrichtung mit ausreichender Geschwindigkeit. Der erfindungsgemäße Sensor ist daher für eine Miniaturisierung und in vivo-Anwendung in physiologischen Flüssigkeiten besonders geeignet.

Die maximale Empfindlichkeit des erfindungsgemäßen Sensors ist von der Affinität zwischen dem Lektin und dem zu untersuchenden Zucker in Gegenwart des hochmolekularen Liganden, von der Empfindlichkeit der Strahlenmeßsonde, der eingeschlossenen Lektinmenge, der Menge des eingeschlossenen Liganden und der spezifischen Radioaktivität der austauschbaren hochmolekularen Sondenmoleküle abhängig. Der optimale Meßbereich kann entsprechend dem Wertebereich der zu erfassenden Zuckerkonzentration durch Wahl des Mengenverhältnisses zwischen dem Lektin und dem hochmolekularen Liganden eingestellt werden, grundsätzlich auch durch die Wahl des Lektins oder des Liganden. Für die Blutzuckerbestimmung kommt als Lektin beispielsweise Concanavallin A, als hochmolekularer Ligand Dextran in Frage. Ist das Dextran mit $^{14}C$ markiert, sollte für die Blutzuckerbestimmung die Dextranmenge zur Lektinmenge in ein solches Verhältnis gebracht werden, daß in Gegenwart von 5 mM Glucose bereits ein signifikanter bzw. gut meßbarer Teil des Dextrans in diffusibler Form vorliegt, der größere Teil jedoch in der(n) lokal begrenzten Zone(n) gebinden bleibt.

Nachfolgend wird die konstruktive Ausführung eines derartigen stereospezifischen Sensors beschrieben:

Das Meßsystem mit der Diffusionskammer wird in einem zylindrischen, etwa 2 mm starken, 8 mm langen Abschnitt eines Kanülendrahtes aus veredeltem Stahl untergebracht, in den seitlich ein Schlitz angebracht wird. Die Wand der Kanüle wird über dem Schlitz gebogen, so daß sie im Querschnitt die Form einer Spirale erhält. In diesen so modifizierten Kanülenabschnitt wird das mehrschichtige Meßsystem eingepaßt und befestigt. Das Meßsystem besteht aus dem Halbleiter-Strahlensensor, einem Diffusionsspalt, einer einseitig mit Polyamid und daran immobilisierten Concanavallin A (100 $\mu g/cm^2$) beschichteten perforierten Abschirmplatte aus Silizium und einer Polysulfonsäuremembran. Die Kammer wird mit einer markierten Dextranlösung gefüllt. Die spezifische Radioaktivität der Dextranlösung wird so gewählt, daß in Gegenwart von 5 mM Glucose ein gut meßbarer Teil der Dextranmoleküle in diffusibler Form vorliegt. Hierdurch erhält man einen Sensor, bei dem die gemessene Radioaktivität von der Glukosekonzentration im mM-Konzentrationsbereich abhängig ist, und bei dem nur ein sehr geringer Bruchteil der ohnehin geringen, weit unter den zulässigen Grenzwerten liegenden $\beta$-Strahlungsintensität nach außen dringt.

## Patentansprüche

1. Stereospezifischer Sensor für die Bestimmung von Zuckern, dadurch gekennzeichnet, daß er eine Diffusionskammer enthält, die Diffusionskammer nach außen mindestens zum Teil von einer für Zucker permeablen, für Makromoleküle undurchlässigen Dialysemembran begrenzt und mit einer Radioaktivitätsmeßsonde verbunden ist, in der Kammer Moleküle eines Zucker-Bindungsproteins, im folgenden als Lektin bezeichnet, und einer unlöslichen oder diffusiblen hochmolekularen Substanz mit Zuckerresten, im folgenden als Ligand bezeichnet, eingeschlossen sind, die Lektinmoleküle eine Affinität für den zu bestimmenden Zucker und für die Zuckerreste des Liganden besitzen, das Lektin oder der Ligand in einer räumlich begrenzten Zone oder mehreren Zonen in der Kammer in unlöslicher Form fixiert ist, und der jeweilige komplementäre hochmolekulare Bindungspartner für das fixierte Lektin oder den fixierten Liganden in austauschbarer und radioaktiv markierter Form vorliegt.

2. Stereospezifischer Sensor nach Anspruch 1, dadurch gekennzeichnet, daß der komplementäre hochmolekulare austauschbare Bindungspartner mit $^{14}$C markiert ist.

3. Stereospezifischer Sensor nach Anspruch 1 und 2, dadurch gekennzeichnet, daß die zum Nachweis der C14-$\beta$-Strahlung geeignete Radioaktivitätsmeßsonde aus einer kompakten, vorzugsweise monolithischen Funktionseinheit besteht, die zur Wandlung der radioaktiven Strahlung, deren Intensität ein Maß für die zu bestimmende Zuckerkonzentration ist, in ein äquivalentes elektrisches, der Strahlungsintensität proportionales Signal einen in Sperrrichtung vorgespannten pn-Übergang in einem halbleitenden Material, vorzugsweise Silizium, und/oder eine Kondensatoranordnung mit einem geeigneten Dielektrikum vorzugsweise $SiO_2$, an die eine Spannung angelegt ist, enthält und in der Schaltungen vorhanden sind, die vorzugsweise mit den Mitteln, der Silizium-CMOS- oder BICMOS-Technologie realisiert und durch geeignete konstruktive Gestaltung vom Strahlungsfeld abgeschirmt, einerseits die für die Wandlung notwendige hohe Spannung intern, auf einen nicht mit der Diffusionskammer in Verbindung stehenden Bereich der Funktionseinheit begrenzt, durch Kaskadenverstärkung einer externen niedrigen Versorgungspannung bereitstellen und andererseits das durch die Wandlung gelieferte elektrische Signal so modifizieren bzw. verstärken, daß zur Weiterverarbeitung, vorzugsweise zur Ansteuerung einer geeigneten Anzeige oder eines die Zuckerkonzentration regulierenden Stellgliedes, nutzbare Signale entstehen.

4. Stereospezifischer Sensor nach Anspruch 1, dadurch gekennzeichnet, daß durch geeignete konstruktive Lösungen, wie zum Beispiel die Anordnung einer korrosionsfesten Abschirmplatte zwischen fixierter Biosubstanz und Strahlungsdetektor oder genügend großem Abstand zwischen der fixierten Biosubstanz und dem Strahlungsdetektor, gewährleistet wird, daß nur über diffusions- oder konvektionsbedingte Verteilung der eingeschlossenen Makromoleküle ein merklicher Anteil Radioaktivität, der der Zuckerkonzentration proportional ist, den Strahlungsdetektor erreichen kann.

5. Stereospezifischer Sensor nach Anspruch 1, 3 und 4 dadurch gekennzeichnet, daß zur Passivierung des strahlungsempfindlichen Teils der Radioaktivitätssonde ein Material benutzt wird, welches biokompatibel ist bzw. mit dem in der Diffusionskammer befindlichen Stoffgemisch chemisch nicht reagiert und daß der genannte strahlungsempfindliche Teil der Radioaktivitätssonde als eine Begrenzung der Diffusionskammer ausgebildet ist.

6. Stereospezifischer Sensor nach Anspruch 1 - 5, dadurch gekennzeichnet, daß der strahlungsempfindliche Teil des Radioaktivitätssensors und die für Zucker permeable, für Makromoleküle undurchlässige Dialysemembran als mechanisch stabile, die Diffusionskammer einschließende Funktionseinheit ausgebildet wird.

7. Stereospezifischer Sensor nach Anspruch 1 - 4, dadurch gekennzeichnet, daß die Kammer aus biokompatiblem Material besteht, das Lektin D-Glukose bindet, und die Menge des eingeschlossenen Lektins so abgestimmt ist, daß in einer annähernd neutral gepufferten wäßrigen Lösung bei einer Glucosekonzentration von 5 mmol/1 bereits ein signifikant meßbarer Teil des radioaktiven hochmolekularen Bindungspartners vom unlöslichen Lektin oder Liganden ausgetauscht und in eine diffusible Form überführt wird.